# EUROPEAN PATENT SPECIFICATION

(11) **EP 3 344 215 B1**
(45) Date of publication and mention of the grant of the patent: **19.05.2021**
(21) Application number: 17734950.3
(22) Date of filing: 23.06.2017
(51) Int. Cl.: A61K 8/24, A61K 8/27, A61K 8/19, A61K 8/365, A61Q 11/00

(54) **ORAL CARE COMPOSITIONS AND METHODS OF USE**
MUNDPFLEGEZUSAMMENSETZUNGEN UND VERFAHREN ZUR VERWENDUNG
COMPOSITIONS DE SOINS BUCCO-DENTAIRES ET LEURS PROCÉDÉS D'UTILISATION

(30) Priority: 24.06.2016 US 201662354269 P
(43) Date of publication of application: 11.07.2018
(73) Proprietor: Colgate-Palmolive Company, New York, NY 10022 (US)
(72) Inventor: THOMSON, Paul, Piscataway, New Jersey 08854 (US); DOGU, Nihal, Dayton, New Jersey 08810 (US); RAJAH, Divino, Long Valley, New Jersey 07853 (US); PRENCIPE, Michael, West Windsor, New Jersey 08550 (US)
(74) Representative: Sonnenhauser, Thomas Martin
(86) International application number: PCT/US2017/038898
(87) International publication number: WO 2017/223389

(56) References cited:
- EP-A1- 0 425 002
- WO-A1-2015/195140
- GB-A- 1 535 031
- US-A- 5 188 820
- US-A1- 2008 138 298

## Description

This invention relates to oral care compositions comprising a first stannous ion source wherein the first source of stannous ions comprises stannous fluoride, a second stannous ion source, wherein the second stannous ion comprises stannous pyrophosphate, and a source of zinc selected from zinc oxide and zinc citrate or zinc lactate, as well as to methods of using and making these compositions.

### BACKGROUND

Oral care compositions present particular challenges in preventing microbial contamination.

Stannous ions, in particular stannous salts such as stannous fluoride, are known anti-microbial agents and are used in various dentifrices as agents for preventing plaque. However, there are certain disadvantages to using stannous salts, such as instability, tendency to stain teeth, astringency, and unpleasant taste for users.

Zinc is also a known antimicrobial agent used in toothpaste compositions. Zinc is a known essential mineral for human health, and has been reported to help strengthen dental enamel and to promote cell repair. Unfortunately, conventional toothpaste formulations often require high concentrations of zinc, e.g., 2% by weight or more, to achieve efficacy. At this concentration, the zinc imparts a notably astringent taste to the composition. There is thus a need for improved antibacterial toothpaste formulations that do not suffer from the drawbacks of conventional compositions.

Accordingly, in view of the drawbacks and disadvantages to using various antimicrobials, such as zinc and stannous, there is a need for oral care compositions with anti-bacterial efficacy, but which are also palatable and desirable for a user.

### BRIEF SUMMARY

It has been surprisingly found that the inclusion of stannous pyrophosphate to an oral care composition containing a stannous source (e.g. stannous fluoride) and a zinc source (e.g. zinc oxide, zinc citrate, zinc lactate, or combinations thereof), selected at certain concentrations and amounts, increased the delivery and availability of stannous and zinc in the oral cavity of a user. The delivery or availability of Zn and Sn is improved in the current invention, where the improvement is relative to comparable products currently on the market.

In one aspect the invention is an oral care composition (Composition 1.0) according to claim 1, comprising:
- A zinc source selected from the group consisting of: zinc oxide, zinc citrate, zinc lactate, and combinations thereof (e.g., ZnO and ZnCit, or e.g., ZnO and ZnLac);
- a first source of stannous (e.g., stannous fluoride); and
- a second source of stannous, wherein the second source of stannous comprises stannous pyrophosphate.

In another embodiment, the invention encompasses compositions for use in a method to improve oral health comprising applying an effective amount of the oral composition according to claim 1 to the oral cavity of a subject in need thereof, e.g., a method to
i. reduce or inhibit formation of dental caries,
ii. reduce, repair or inhibit early enamel lesions, e.g., as detected by quantitative light- induced fluorescence (QLF) or electrical caries measurement(ECM),
iii. reduce or inhibit demineralization and promote remineralization of the teeth,
iv. reduce hypersensitivity of the teeth,
v. reduce or inhibit gingivitis,
vi. promote healing of sores or cuts in the mouth,
vii. reduce levels of acid producing bacteria,
viii. to increase relative levels of arginolytic bacteria,
ix. inhibit microbial bio film formation in the oral cavity,
x. raise and/or maintain plaque pH at levels of at least pH 5.5 following sugar challenge,
xi. reduce plaque accumulation,
xii. treat dry mouth,
xiii. enhance systemic health, including cardiovascular health,e.g., by reducing potential for systemic infection via the oral tissues,
xiv. Whiten teeth,
xv. reduce erosion of the teeth,
xvi. immunize (or protect) the teeth against cariogenic bacteria and their effects, and/or
xvii. clean the teeth and oral cavity.

The invention further comprises the use of sodium bicarbonate, sodium methyl cocoyl taurate (tauranol), MIT, and benzyl alcohol and combinations thereof in the manufacture of a Composition of the Invention, e.g., for use in any of the indications set forth in the above method of Composition 1.0, *et seq.*

### DETAILED DESCRIPTION

As used herein, the term "oral composition" means the total composition that is delivered to the oral surfaces. The composition is further defined as a product which, during the normal course of usage, is not, the purposes of systemic administration of particular therapeutic agents, intentionally swallowed but is rather retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively the oral composition may be dual phase dispensed from a separated compartment dispenser.

### Stannous Ion Source

In some embodiments, the first stannous source comprises a stannous source selected from stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide, or mixtures thereof. In some embodiments, the first stannous source comprises stannous fluoride.

### Fluoride Ion Source

The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al. Representative fluoride ion sources used with the present invention (e.g., Composition 1.0 *et seq*.) include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. Where the formulation comprises calcium salts, the fluoride salts are preferably salts wherein the fluoride is covalently bound to another atom, e.g., as in sodium monofluorophosphate, rather than merely ionically bound, e.g., as in sodium fluoride.

### Surfactants

The invention may in some embodiments contain anionic surfactants, e.g., the Compositions of Composition 1.0, *et seq.,* for example, water-soluble salts of higher fatty acid monoglyceride monosulfates, such as the sodium salt of the monosulfated monoglyceride of hydrogenated coconut oil fatty acids such as sodium N- methyl N-cocoyl taurate, sodium cocomo-glyceride sulfate; higher alkyl sulfates, such as sodium lauryl sulfate; higher alkyl-ether sulfates, e.g., of formula CH₃(CH₂)ₘCH₂(OCH₂CH₂)ₙOS0₃X, wherein m is 6-16, e.g., 10, n is 1-6, e.g., 2, 3 or 4, and X is Na or, for example sodium laureth-2 sulfate (CH₃(CH2)₁₀CH₂(OCH₂CH₂)₂OS0₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2- ethyl laurate potassium sulfoacetamide) and sodium lauryl sarcosinate. By "higher alkyl" is meant, e.g., C₆-₃₀ alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %, and optimal concentrations depend on the particular formulation and the particular surfactant. In one embodiment, the anionic surfactant is present at from 0.03% to 5% by weight, e.g., 1.5%.

In another embodiment, cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al., herein incorporated by reference. Certain cationic surfactants can also act as germicides in the compositions.

Illustrative nonionic surfactants of Composition 1.0, *et seq.,* that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

In another embodiment illustrative zwitterionic surfactants of Composition 1.0, *et seq*., that can be used in the compositions of the invention include betaines (such as cocamidopropylbetaine), derivatives of aliphatic secondary and tertiary amines in which the aliphatic radical can be a straight or branched chain and wherein one of the aliphatic substituents contains about 8-18 carbon atoms and one contains an anionic water-solubilizing group (such as carboxylate, sulfonate, sulfate, phosphate or phosphonate), and mixtures of such materials.

The surfactant or mixtures of compatible surfactants can be present in the compositions of the present invention in 0.1% to 5%, in another embodiment 0.3% to 3% and in another embodiment 0.5% to 2% by weight of the total composition.

### Flavoring Agents

The oral care compositions of the invention may also include a flavoring agent. Flavoring agents which are used in the practice of the present invention include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.01 to 1% by weight.

### Chelating and anti-calculus agents

The oral care compositions of the invention also may include one or more chelating agents able to complex calcium found in the cell walls of the bacteria. Binding of this calcium weakens the bacterial cell wall and augments bacterial lysis.

Another group of agents suitable for use as chelating or anti-calculus agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 0.1 wt. % pyrophosphate ions, e.g., 0.1 to 3 wt 5, e.g., 0.1 to 2 wt %, e.g., 0.1 to 1 wt%, e.g., 0.2 to 0.5 wt%. The pyrophosphates also contribute to preservation of the compositions by lowering water activity.

In various embodiments of the present disclosure (e.g., Composition 1.0 *et seq*), the compositions further comprise one or more anticalculus (tartar control) agents. Suitable anticalculus agents include without limitation mono-phosphates (e.g. monobasic, dibasic or tribasic phosphate) and P1-6 polyphosphates (e.g., pyrophosphates, tripolyphosphate, tetraphosphates and hexametaphosphate salts, zinc salts (e.g., zinc citrate, zinc chloride, zinc citrate trihydrate), Gantrez® (a copolymer of methylvinyl ether (PVM) and maleic acid (MA)), polyaminopropanesulfonic acid (AMPS), polypeptides, polyolefin sulfonates, polyolefin phosphates, and diphosphonates. In certain embodiments, the other anticalculus agents are alkali and/or alkaline earth metal phosphate salts, for example, sodium, potassium or calcium salts. In certain embodiments, the composition includes mono-phosphates (e.g. monobasic, dibasic or tribasic phosphate), P1-6 polyphosphates, Gantrez, or a combination thereof. Still in certain embodiments, the composition includes sodium tripolyphosphate, tetrasodium pyrophosphate, Gantrez, or a combination thereof.

### Polymers

The oral care compositions of the invention also optionally include one or more polymers, such as polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Certain embodiments include 1 :4 to 4: 1 copolymers of maleic anhydride or acid with another polymerizable ethylenically unsaturated monomer, for example, methyl vinyl ether (methoxyethylene) having a molecular weight (M.W.) of about 30,000 to about 1,000,000. These copolymers are available for example as Gantrez AN 139(M.W. 500,000), AN 1 19 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1 : 1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid.

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, xanthan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.5% to about 5.0% by weight of the total composition are used.

### Abrasives

Natural calcium carbonate is found in rocks such as chalk, limestone, marble and travertine. It is also the principle component of egg shells and the shells of mollusks. The natural calcium carbonate abrasive of the invention is typically a finely ground limestone which may optionally be refined or partially refined to remove impurities. For use in the present invention, the material has an average particle size of less than 10 microns, e.g., 3-7 microns, e.g. about 5.5 microns. For example a small particle silica may have an average particle size (D50) of 2.5 - 4.5 microns. Because natural calcium carbonate may contain a high proportion of relatively large particles of not carefully controlled, which may unacceptably increase the abrasivity, preferably no more than 0.01%, preferably no more than 0.004% by weight of particles would not pass through a 325 mesh. The material has strong crystal structure, and is thus much harder and more abrasive than precipitated calcium carbonate. The tap density for the natural calcium carbonate is for example between 1 and 1.5 g/cc, e.g., about 1.2 for example about 1.19 g/cc. There are different polymorphs of natural calcium carbonate, e.g., calcite, aragonite and vaterite, calcite being preferred for purposes of this invention. An example of a commercially available product suitable for use in the present invention includes Vicron ® 25-11 FG from GMZ.

Precipitated calcium carbonate is generally made by calcining limestone, to make calcium oxide (lime), which can then be converted back to calcium carbonate by reaction with carbon dioxide in water. Precipitated calcium carbonate has a different crystal structure from natural calcium carbonate. It is generally more friable and more porous, thus having lower abrasivity and higher water absorption. For use in the present invention, the particles are small, e.g., having an average particle size of 1 - 5 microns, and e.g., no more than 0.1 %, preferably no more than 0.05% by weight of particles which would not pass through a 325 mesh. The particles may for example have a D50 of 3-6 microns, for example 3.8=4.9, e.g., about 4.3; a D50 of 1-4 microns, e.g. 2.2-2.6 microns, e.g., about 2.4 microns, and a D10 of 1-2 microns, e.g., 1.2-1.4, e.g. about 1.3 microns. The particles have relatively high water absorption, e.g., at least 25 g/100g, e.g. 30-70 g/100g. Examples of commercially available products suitable for use in the present invention include, for example, Carbolag® 15 Plus from Lagos Industria Quimica.

In certain embodiments the invention may comprise additional calcium-containing abrasives, for example calcium phosphate abrasive, e.g., tricalcium phosphate (Ca₃(P0₄)₂), hydroxyapatite (Ca₁₀(P0₄)₆(OH)₂), or dicalcium phosphate dihydrate (CaHP0₄ · 2H₂0, also sometimes referred to herein as DiCal) or calcium pyrophosphate, and/or silica abrasives, sodium metaphosphate, potassium metaphosphate, aluminum silicate, calcined alumina, bentonite or other siliceous materials, or combinations thereof. Any silica suitable for oral care compositions may be used, such as precipitated silicas or silica gels. For example synthetic amorphous silica. Silica may also be available as a thickening agent, e.g., particle silica. For example, the silica can also be small particle silica (e.g., Sorbosil AC43 from PQ Corporation, Warrington, United Kingdom). However the additional abrasives are preferably not present in a type or amount so as to increase the RDA of the dentifrice to levels which could damage sensitive teeth, e.g., greater than 130.

### Water

Water is present in the oral compositions of the invention (e.g., Composition 1.0 *et seq*). Water, employed in the preparation of commercial oral compositions should be deionized and free of organic impurities. Water commonly makes up the balance of the compositions and includes 5% to 45%, e.g., 10% to 20%, e.g., 25 - 35%, by weight of the oral compositions. This amount of water includes the free water which is added plus that amount which is introduced with other materials such as with sorbitol or silica or any components of the invention. The Karl Fischer method is a one measure of calculating free water.

### Humectants

Within certain embodiments of the oral compositions (e.g. Composition 1.0 *et seq*), it is also desirable to incorporate a humectant to reduce evaporation and also contribute towards preservation by lowering water activity. Certain humectants can also impart desirable sweetness or flavor to the compositions. The humectant, on a pure humectant basis, generally includes 15% to 70% in one embodiment or 30% to 65% in another embodiment by weight of the composition.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the compositions herein.

The present invention in its method aspect involves applying to the oral cavity a safe and effective amount of the compositions described herein.

The compositions and methods according to the invention (e.g., Composition 1.0 *et seq*) can be incorporated into oral compositions for the care of the mouth and teeth such as toothpastes, transparent pastes, gels, mouth rinses, sprays and chewing gum.

As used throughout, ranges are used as shorthand for describing each and every value that is within the range. Any value within the range can be selected as the terminus of the range. In addition, all references cited herein are hereby incorporated by reference in their entireties. In the event of a conflict in a definition in the present disclosure and that of a cited reference, the present disclosure controls. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible without departing from the spirit and scope thereof. Various modifications of the invention in addition to those shown and described herein should be apparent to those skilled in the art and are intended to fall within the appended claims.

### Example 1

**Dentifrice Formulation A**

| **Ingredient** | **Weight %** |
|---|---|
| DEMINERALIZED WATER | Q. S. |
| CITRIC ACID - ANHYDROUS U SP, EP | 0.60 |
| TRISODIUM CITRATE DIHYDRATE - USP | 1.5 |
| STANNOUS FLUORIDE, USP | 0.454 |
| ZINC OXIDE | 1.0 |
| ZINC L-LACTATE DIHYDRATE | 0.85 |
| 99.0% - 101.0% GLYCERIN - USP, EP VEG | 42.3 |
| POLYETHYLENE GLYCOL 600 | 3.0 |
| PROPYLENE GLYCOL USP, EP | 4.0 |
| THICKENERS | 6.4 |
| POLYVINYL PYRROLIDONE | 1.25 |
| STANNOUS PYROPHOSPHATE | 1.0 |
| ABRASIVES | 19.0 |
| TETRASODIUM PYROPHOSPHATE - FINE FCC | 2.0 |
| ANIONIC SURFACTANT | 1.75 |
| ZWITTERIONIC SURFACTANT | 1.0 |
| FLAVORS, COLORS AND SWEETENERS | 2.15 |
| PVM/MA COPOLYMER | 0.606 |
| SODIUM PHOSPHATE, TRIBASIC, 12-HYDRATE | 1.0 |
| PHOSPHORIC ACID 85% REAGENT GRADE | 0.50 |
| TOTAL COMPONENTS | 100.0 |

### Example 2

**Dentifrice Formulation B**

| **Ingredient** | **Weight %** |
|---|---|
| DEMINERALIZED WATER | Q.S. |
| CITRIC ACID - ANHYDROUS USP, EP | 0.6 |
| TRISODIUM CITRATE DIHYDRATE - USP | 1.5 |
| STANNOUS FLUORIDE, USP | 0.454 |
| ZINC OXIDE | 1.0 |
| ZINC CITRATE TRIHYDRATE | 0.5 |
| 99.0% - 101.0% GLYCERIN - USP, EP VEG | 42.84 |
| POLYETHYLENE GLYCOL 600 | 3.0 |
| PROPYLENE GLYCOL USP, EP | 4.0 |
| THICKENERS | 6.5 |
| POLYVINYL PYRROLIDONE | 1.25 |
| STANNOUS PYROPHOSPHATE | 1.0 |
| ABRASIVES | 19.0 |
| TETRASODIUM PYROPHOSPHATE - FINE FCC | 2.0 |
| ANIONIC SURFACTANT | 1.75 |
| ZWITTERIONIC SURFACTANT | 1.0 |
| FLAVORS, COLORS AND SWEETENERS | 2.15 |
| GANTREZ S-97 (B.F.) - HIGH SOLIDS SOLN | 0.606 |
| SODIUM PHOSPHATE, TRIBASIC, 12-HYDRATE | 1.0 |
| PHOSPHORIC ACID 85% REAGENT GRADE | 0.25 |
| TOTAL COMPONENTS | 100.0 |

### Example 3

*In vitro* assay for monitoring metal ion deposition

Test Formulations with
a. Dentifrice Formulation A: 0.454% SnF₂, 1.0% ZnO and 0.5% Zn lactate, and 2% Tetrasodium Pyrophosphate (TSPP), 2.1% Citrate Buffer (comprising 1.5% sodium citrate and 0.6% citric acid) and 1% Stannous Pyrophosphate (Listed in Example 1)
b. Dentifrice Formulation C: 0.454% SnF₂, 1.0% ZnO and 0.5% Zn Citrate, and 2% Sodium Tripolyphosphate (STPP), 2.1 %, Citrate Buffer (comprising 1.5% sodium citrate and 0.6% citric acid) and 1% Stannous Pyrophosphate;
demonstrate improved metal deposition, in *in vitro* bovine uptake assays when compared to control formulations which contain 0.454% SnF₂ and 2.5% zinc lactate, but which do not contain stannous pyrophosphate:

**Table 1**

| | Total Stannous Metal Deposition (ppm) | Total Zinc Metal Deposition (ppm) |
|---|---|---|
| Control Formulation (0.454 wt% SnF₂, and 2.5 wt.% zinc lactate) | 0.74 | 1.69 |
| Dentifrice Formulation A | 1.30 | 4.22 |
| Dentifrice Formulation C | 0.80 | 6.17 |

Dentifrice Formulation C is also compared to formulations with the following backbone: 0.454% SnF₂, 1.0% ZnO and 0.5% Zn Citrate, 3% STPP, and 3.6% Citrate Buffer (3.0% trisodium citrate dihydrate, 0.6% citric acid). There is roughly a 32% increase in the amount of soluble zinc in the Formulation C samples, with comparable tin levels in both samples.

### Example 4

### In vitro ATP assay

In order to screen for preliminary efficacy, the dual stannous dentifrice is subjected to an ATP- bacteria viability test. This method determines the number of viable bacterial cells in culture based on the quantification of ATP present. ATP is an indicator of metabolically active cells. This method uses a luciferase reporter to measure the amount of cellular ATP present, hence the more effective the dentifrice is, the less ATP found.

In the ATP assay, Dentifrice Formulation A (detailed in Example 1) shows, roughly, greater than 80% reduction in bacterial cell viability when compared to control formulations which contain 0.454% SnF₂ and 2.5% zinc lactate, but which do not contain stannous pyrophosphate.

### Example 5

### ESCA Uptake assay

The purpose of the Uptake assay is to determine the percentage of metal ion uptake on the surface of bovine enamel after pellicle growth, 1% citric acid challenge, and treatment of a test dentifrice (Dentifrice Z) as detailed in the below Table 2.

**Table 2**

| | Sn (Atomic %) | Ratio of Sn on Enamel Surface | Zn (Atomic %) | Ratio of Zn on Enamel Surface |
|---|---|---|---|---|
| Positive Control Formulation (0.454% SnF, 1% ZnO, 0.5% Zn citrate and no Stannous Pyrophoshpate) | 0.12% | 1 | 1.4% | 1 |
| Dentifrice Formulation Z (0.454% SnF, 1% ZnO, 0.5% Zn citrate, and 1% Stannous Pyrophosphate | 0.3 | 1.61 | 2.5% | 1.15 |

| | | | | |
|---|---|---|---|---|
| (Values are normalized to "1") | | | | |

Bovine enamel is treated with saliva for 2 hrs, subject to an acid etch, and treated with dentifrice slurry. Specifically, saliva is removed and the bovine enamel is subject to 1% citric acid treatment and subsequently rinsed with deionized water. One group is treated with deionized water in order to establish a baseline. The subsequent test groups are treated with a 1:2 ratio of dentrifrice and deionized water slurry then rinsed with deionized water. Bovine enamel are then subject to ESCA analysis.

### Example 6

### Representative Formula 1

| **Ingredient** | **Weight %** |
|---|---|
| DEMINERALIZED WATER | Q.S. |
| CITRIC ACID - ANHYDROUS USP, EP | 0.6 |
| TRISODIUM CITRATE DIHYDRATE - USP | 1.5 |
| STANNOUS FLUORIDE, USP | 0.454 |
| ZINC OXIDE | 1 |
| ZINC CITRATE TRIHYDRATE | 0.5 |
| 99.5% VEG REFINED GLYCERIN | 42.74 |
| POLYETHYLENE, GLYCOL 600 | 3 |
| PROPYLENE GLYCOL USP, EP | 4 |
| Microcrystalline cellulose/Sodium CMC | 1 |
| Polyvinyl pyrrolidone | 1.25 |
| STANNOUS PYROPHOSPHATE | 1 |
| Abrasives | 19 |
| TETRASODIUM PYROPHOSPHATE - FINE FCC | 2 |
| Anionic Surfactant | 1.75 |
| Zwitterionic Surfactant | 1 |
| Flavors, Colors and Sweeteners | 3.2 |
| GANTREZ S-97 (B.F.) - HIGH SOLIDS SOLN | 0.606 |
| Sodium Phosphate,Tribasic, 12-Hydrate | 1 |
| Phosphoric acid 85% Reagent Grade | 0.25 |
| SYNTHETIC AMORPHOUS SILICA | 5 |
| Total Components | 100 |

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts iven are based on the active weight of the material.

## Claims

1. An oral care composition comprising:
a zinc source comprising 0.75-1.25 wt.% zinc oxide and 0.25-1.0 wt.% zinc citrate, or 0.5-0.9 wt.% zinc lactate, based on the weight of the oral composition;
a first source of stannous ions, wherein the first source of stannous ions comprises stannous fluoride in an amount of about 0.45 wt.%, based on the weight of the oral composition; and
a second source of stannous, wherein the second source of stannous comprises stannous pyrophosphate in an amount of 0.1-3 wt.%, based on the weight of the oral composition.

2. The oral care composition of claim 1, wherein the ratio of the amount of zinc oxide to zinc citrate is from 1.5:1 to 4.5:1.

3. The oral care composition of claim 1, wherein the ratio of the amount of zinc oxide to zinc lactate is from 1.2:1 to 4.5:1.

4. The oral care composition of any preceding claims, wherein the composition comprises one or more alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, disodium hydrogen orthophosphate, monosodium phosphate, pentapotassium triphosphate and mixtures thereof.

5. The oral care composition of claim 4, wherein the composition comprises an alkali phosphate salt in an amount of from 1-20% by weight, based on the total weight of the composition.

6. The oral care composition of claim 4, wherein the alkali phosphate salt is tetrapotassium pyrophosphate.

7. The oral care composition of claim 4, wherein the alkali phosphate salt is sodium tripolyphosphate.

8. A composition of any of the preceding claims, wherein the composition comprises:
a. Zinc Oxide at about 1.0 wt.%;
b. Zinc Citrate at about 0.5 wt%;
c. Stannous pyrophosphate at about 1.0 wt%;
d. and further comprises tetrasodium pyrophosphate at about 2.0 wt%; and
e. optionally, further comprising a citrate buffer system, wherein the buffer system comprises tri-sodium citrate and citric acid.

9. A composition of any of claims 1-7, wherein the composition comprises:
a. Zinc Oxide at about 1.0 wt.%;
b. Zinc lactate at about 0.5 wt%;
c. Stannous pyrophosphate at about 1.0 wt%;
d. and further comprises Sodium tripolyphosphate at about 2.0 wt%,
e. optionally, further comprising a citrate buffer system, wherein the buffer system comprises tri-sodium citrate and citric acid.

10. The composition of any of the preceding claims, wherein the composition may be any of the following selected from: a toothpaste, transparent paste, gel, mouth rinse, spray and chewing gum.

11. Oral composition of any of the preceding claims for use in a method to improve oral health comprising applying an effective amount of said oral composition, wherein the oral care composition is applied to the oral cavity of a subject in need thereof.

## Patentansprüche

1. Mundpflegezusammensetzung, umfassend:
eine Zinkquelle, umfassend 0,75-1,25 Gew.-% Zinkoxid und 0,25-1,0 Gew.-% Zinkcitrat oder 0,5-0,9 Gew.-% Zinklactat, bezogen auf das Gewicht der oralen Zusammensetzung;
eine erste Zinnionen-Quelle, wobei die erste Zinnionen-Quelle Zinnfluorid in einer Menge von etwa 0,45 Gew.-%, bezogen auf das Gewicht der oralen Zusammensetzung, umfasst; und
eine zweite Zinnquelle, wobei die zweite Zinnquelle Zinnpyrophosphat in einer Menge von 0,1 bis 3 Gew.-%, bezogen auf das Gewicht der oralen Zusammensetzung, umfasst.

2. Mundpflegezusammensetzung nach Anspruch 1, wobei das Verhältnis der Menge an Zinkoxid zu Zinkcitrat von 1,5:1 bis 4,5:1 beträgt.

3. Mundpflegezusammensetzung nach Anspruch 1, wobei das Verhältnis der Menge an Zinkoxid zu Zinklactat von 1,2:1 bis 4,5:1 beträgt.

4. Mundpflegezusammensetzung nach einem beliebigen der vorhergehenden Ansprüche, wobei die Zusammensetzung ein oder mehrere Alkaliphosphatsalze, ausgewählt aus Natriumphosphat dibasisch, Kaliumphosphat dibasisch, Dicalciumphosphatdihydrat, Calciumpyrophosphat, Tetranatriumpyrophosphat, Tetrakaliumpyrophosphat, Natriumtripolyphosphat, Dinatriumhydrogenorthophosphat, Mononatriumphosphat, Pentakaliumtriphosphat und Mischungen davon, umfasst.

5. Mundpflegezusammensetzung nach Anspruch 4, wobei die Zusammensetzung ein Alkaliphosphatsalz in einer Menge von 1-20 Gewichts-%, bezogen auf das Gesamtgewicht der Zusammensetzung, umfasst.

6. Mundpflegezusammensetzung nach Anspruch 4, wobei das Alkaliphosphatsalz Tetrakaliumpyrophosphat ist.

7. Mundpflegezusammensetzung nach Anspruch 4, wobei das Alkaliphosphatsalz Natriumtripolyphosphat ist.

8. Zusammensetzung nach einem beliebigen der vorhergehenden Ansprüche, wobei die Zusammensetzung umfasst:
a. Zinkoxid zu etwa 1,0 Gew.-%;
b. Zinkcitrat zu etwa 0,5 Gew.-%;
c. Zinnpyrophosphat zu etwa 1,0 Gew.-%;
d. und weiterhin Tetranatriumpyrophosphat zu etwa 2,0 Gew.-% umfasst; und
e. gegebenenfalls weiterhin umfassend ein Citratpuffersystem, wobei das Puffersystem Trinatriumcitrat und Zitronensäure umfasst.

9. Zusammensetzung nach einem beliebigen der Ansprüche 1-7, wobei die Zusammensetzung umfasst:
a. Zinkoxid zu etwa 1,0 Gew.-%;
b. Zinklactat zu etwa 0,5 Gew.-%;
c. Zinnpyrophosphat zu etwa 1,0 Gew.-%;
d. und weiterhin Natriumtripolyphosphat zu etwa 2,0 Gew.-% umfasst,
e. gegebenenfalls weiterhin umfassend ein Citratpuffersystem, wobei das Puffersystem Trinatriumcitrat und Zitronensäure umfasst.

10. Zusammensetzung nach einem beliebigen der vorhergehenden Ansprüche, wobei die Zusammensetzung eine der folgenden sein kann, ausgewählt aus: einer Zahnpasta, transparenter Paste, Gel, Mundspülung, Spray und Kaugummi.

11. Orale Zusammensetzung nach einem beliebigen der vorhergehenden Ansprüche zur Verwendung in einem Verfahren um die orale Gesundheit zu verbessern, umfassend das Auftragen einer wirksamen Menge der oralen Zusammensetzung, wobei die Mundpflegezusammensetzung in die Mundhöhle eines Subjekts, das diese benötigt, aufgetragen wird.

## Revendications

1. Composition de soins bucco-dentaires comprenant :
une source de zinc comprenant 0,75 à 1,25 % en poids d'oxyde de zinc et 0,25 à 1,0 % en poids de citrate de zinc, ou 0,5 à 0,9 % en poids de lactate de zinc, par rapport au poids de la composition orale ;
une première source d'ions stanneux, dans laquelle la première source d'ions stanneux comprend du fluorure stanneux en une quantité d'environ 0,45 % en poids, par rapport au poids de la composition orale ; et
une seconde source de stanneux, dans laquelle la seconde source de stanneux comprend du pyrophosphate stanneux en une quantité de 0,1 à 3 % en poids, par rapport au poids de la composition orale.

2. Composition de soins bucco-dentaires selon la revendication 1, dans laquelle le rapport de la quantité d'oxyde de zinc au citrate de zinc est de 1,5:1 à 4,5:1.

3. Composition de soins bucco-dentaires selon la revendication 1, dans laquelle le rapport de la quantité d'oxyde de zinc au lactate de zinc est de 1,2:1 à 4,5:1.

4. Composition de soins bucco-dentaires selon l'une quelconque des revendications précédentes, dans laquelle la composition comprend un ou plusieurs sels de phosphate alcalin choisis parmi le phosphate de sodium dibasique, le phosphate de potassium dibasique, le phosphate dicalcique dihydraté, le pyrophosphate de calcium, le pyrophosphate tétrasodique, le pyrophosphate tétrapotassique, le tripolyphosphate de sodium, l'hydrogénophosphate disodique, le phosphate monosodique, le triphosphate de pentapotassium et leurs mélanges.

5. Composition de soins bucco-dentaires selon la revendication 4, dans laquelle la composition comprend un sel de phosphate alcalin en une quantité de 1 à 20 % en poids, par rapport au poids total de la composition.

6. Composition de soins bucco-dentaires selon la revendication 4, dans laquelle le sel de phosphate alcalin est le pyrophosphate de tétrapotassium.

7. Composition de soins bucco-dentaires selon la revendication 4, dans laquelle le sel de phosphate alcalin est le tripolyphosphate de sodium.

8. Composition selon l'une quelconque des revendications précédentes, dans laquelle la composition comprend :
a. de l'oxyde de zinc à environ 1,0 % en poids ;
b. du citrate de zinc à environ 0,5 % en poids ;
c. du pyrophosphate stanneux à environ 1,0 % en poids ;
d. et comprend en outre du pyrophosphate tétrasodique à environ 2,0 % en poids ; et
e. éventuellement, comprenant en outre un système tampon citrate, dans lequel le système tampon comprend du citrate trisodique et de l'acide citrique.

9. Composition selon l'une quelconque des revendications 1 à 7, dans laquelle la composition comprend :
a. de l'oxyde de zinc à environ 1,0 % en poids ;
b. du lactate de zinc à environ 0,5 % en poids ;
c. du pyrophosphate stanneux à environ 1,0 % en poids ;
d. et comprend en outre du tripolyphosphate de sodium à environ 2,0 % en poids ;
e. éventuellement, comprenant en outre un système tampon citrate, dans lequel le système tampon comprend du citrate trisodique et de l'acide citrique.

10. Composition selon l'une quelconque des revendications précédentes, dans laquelle la composition peut être l'une quelconque des suivantes, choisie parmi : un dentifrice, une pâte transparente, un gel, un rince bouche, un aérosol et une gomme à mâcher.

11. Composition orale selon l'une quelconque des revendications précédentes pour une utilisation dans un procédé pour améliorer la santé bucco-dentaire comprenant l'application d'une quantité efficace de ladite composition orale, dans laquelle la composition de soins bucco-dentaires est appliquée à la cavité buccale d'un sujet en ayant besoin.
